# EUROPEAN PATENT APPLICATION

(11) **EP 4 825 331 A2**
(43) Date of publication of application: **21.10.2026**
(21) Application number: 26201388.1
(22) Date of filing: 05.09.2019
(51) Int. Cl.: A61B 18/14

(54) **ELECTROSURGICAL GENERATOR VERTIFICATION SYSTEM**

(30) Priority: 05.09.2018 US 201862727176 P
(62) Document number(s) of the earlier application(s) in accordance with Art. 76 EPC: 24207068.8 / 4 470 492; 19772930.4 / 3 846 709
(71) Applicant: Applied Medical Resources Corporation, Rancho Santa Margarita, CA 92688 (US)
(72) Inventor: ADL, Sean, Rancho Santa Margarita, 92688 (US); SOTO, Andrew, Rancho Santa Margarita, 92688 (US); GORIN, Igor, Rancho Santa Margarita, 92688 (US)
(74) Representative: Dolleymores

(57) **Abstract**

Systems and methods for performing a self-verification system test upon activation of an electrosurgical generator are described. The systems and methods allow for enhancing surgical outcomes by providing generators having accurate RF energy generation, measurement, calibration and self-testing system. This is achieved through implementation of an automated self-verification process at a power start-up of the generator, which allows for rapidly identifying a potential generator issue prior to any use of a connected electrosurgical instrument or supply of any RF energy to the tissue or vessel through the electrosurgical instrument. Additionally, one or more internal impedance loads are integrated within the electrosurgical generator. The internal impedance loads with multiple configurations are utilized to verify the voltage, current, power, and/or phase measurements of the generator. By incorporating or integrating the self-verification process and its related hardware resources within the electrosurgical generator, many improvements in outcome of pre-surgical procedures may be achieved.

## Description

### CROSS-REFERENCE TO RELATED APPLICATIONS

This application claims the benefit of both co-pending U.S. Provisional Application Serial No. 62/727,176 filed on September 5, 2018, and U.S. Provisional Application Serial No. 62/727,195 filed on September 5, 2018, which are hereby expressly incorporated by reference in their entirety for all purposes.

### TECHNICAL FIELD

The present disclosure is generally directed to electrosurgical generator systems and methods and more particularly to output verification of electrosurgical generators configured to supply radiofrequency (RF) energy.

### BACKGROUND

Electrosurgical devices or instruments have become available that use radiofrequency (RF) energy to perform certain surgical tasks such as coagulate, fuse, or cut tissue. Such electrosurgical instruments typically fall within two classifications: monopolar and bipolar. In monopolar instruments, electrical energy is supplied to one or more electrodes on the instrument with high current density while a separate return electrode is electrically coupled to a patient and is often designed to minimize current density. Bipolar electrosurgical instruments, which operate without separate return electrodes, can deliver electrical signals to a focused tissue area with reduced risks.

Even with the relatively focused surgical effects of bipolar electrosurgical instruments, surgical outcomes are often highly dependent on surgeon skill. Enhanced generators have been made to reduce this dependency. However, such generators have had shortcomings in that they can provide inconsistent results in determining tissue coagulation, fusion, or cutting endpoints for varied tissue types or combined tissue masses. These systems can also fail to provide consistent electrosurgical results among use of different instruments; having different instrument and electrode geometries and subjected to different tissue types and tissue amounts. Some of these shortcomings are sometimes exacerbated by inaccurate RF energy generation, measurement, calibration and testing of such systems. Unavailable or unreliable test equipment and/or unavailable or inexperienced personnel also contribute to these shortcomings or do little to assist in overcoming these shortcomings. Hence, embodiments of the present invention are intended to obviate or at least alleviate the aforementioned problems of the conventional electrosurgical generators.

### SUMMARY

In accordance with various embodiments, an electrosurgical system for sealing, fusing and/or cutting tissue is provided. The electrosurgical system comprises an electrosurgical generator and an electrosurgical instrument or device. The electrosurgical generator in various embodiments may include an RF amplifier, a feedback system and a primary controller. The RF amplifier supplies RF energy through a removably coupled electrosurgical instrument configured to fuse, seal and/or cut tissue. The primary controller is arranged to initiate and halt the supply of RF energy and the feedback system is arranged to monitor the supplied RF energy. By monitoring or verifying the electrical properties of the RF output, the electrosurgical generator of the present invention, in accordance with various embodiments, ensures the RF energy being outputted is optimal for surgical treatment of tissue or vessels.

In accordance with one aspect of the present invention, an electrosurgical system for performing surgical procedures is provided. The electrosurgical system may include an electrosurgical generator adapted to perform a self-verification system test upon activation of the generator and a plurality of internal impedance loads. The electrosurgical generator includes a processor which is configured to cause initiating the self-verification system test after determining a predetermined period of time has elapsed after starting the generator. If a failure of the generator is not detected, the processor allows for supplying RF energy to a connected electrosurgical hand device. If a failure of the generator is detected by the self-verification system test for a number of consecutive times, the processor generates a system error and notifies the user or surgeon of the generator's error.

In accordance with a second aspect of the present invention, a method for performing an auto-verification and self-verification system of an electrosurgical generator prior to performing surgical procedure is provided. The method includes the steps of: initiating a self-verification system test after determining a predetermined period of time has elapsed upon activation of the generator; setting RF regulation modes and RF resolution settings after initiating the self-verification system test; generating RF energy and directing RF output to a plurality of impedance loads within the generator; determining whether the self-verification system test has been completed; and recording self-verification system test completion timestamp upon completion of the self-verification system test. After completion of the self-verification system test, the method further includes a step of determining whether a generator failure has occurred, and accordingly, allows for initiating or halting the supply of RF energy to a connected electrosurgical instrument.

In accordance with a third aspect of the present invention, a control system for use with an electrosurgical generator adapted to perform a self-verification system test upon activation of the generator is provided. The control system may include an RF amplifier for supplying RF energy, a feedback system for continually monitoring electrical properties of the supplied RF energy and generating digital RF signals relating thereto and a primary microcontroller configured to initiate the self-verification system test and to allow supplying and halting the supplied RF energy to a connected electrosurgical hand device based at least in part on results of the self-verification system test.

In accordance with a fourth aspect of the present invention, there is provided an electrosurgical system for performing surgical procedures. The electrosurgical system may include an electrosurgical generator adapted to supply electrosurgical RF energy to tissue and an electrosurgical instrument comprising at least one active electrode adapted to apply the supplied electrosurgical RF energy to tissue. The electrosurgical generator may include a microprocessor programmed to regulate the RF output of the generator to a predetermined RF regulation value across a plurality of RF regulation modes and a plurality of RF resolution settings. The electrosurgical generator further includes a feedback system configured to measure electrical properties of the RF output supplied to one or more internal impedance loads across a plurality of channels. The microprocessor is further programed to compare all measurements values of the feedback system channels for each of the plurality of RF regulation modes and RF resolution settings and determine whether a fault condition exist within the generator.

In accordance with a fifth aspect of the present invention, an electrosurgical generator is provided. The electrosurgical generator may include a plurality of impedance loads integrated within an RF amplifier that supplies RF energy and a feedback system measuring the electrical properties of RF energy directed to the plurality of impedance loads across a plurality of channels. The electrosurgical generator further includes a primary microcontroller configured to initiate a self-verification system test upon activation of the generator to verify the RF output of the generator across a plurality of RF regulation modes and RF resolution setting for determining whether a generator failure exist.

In accordance with various embodiments, an electrosurgical generator is provided. The generator comprises a plurality of switchable impedance loads integrated within the generator and through which RF energy is supplied there through and a microcontroller configured to switch in one or more of the plurality of switchable impedance loads to verify RF output of the generator.

Many of the attendant features of the present inventions will be more readily appreciated as the same becomes better understood by reference to the foregoing and following description and considered in connection with the accompanying drawings.

### BRIEF DESCRIPTION OF THE DRAWING

The present disclosure is described in conjunction with the appended figures:
FIG. 1 is a perspective view of an electrosurgical generator in accordance with various embodiments of the present invention.
FIG. 2 is a perspective view of an electrosurgical hand device in accordance with various embodiments of the present invention.
FIG. 3 is a perspective view of an alternative embodiment of an electrosurgical hand device in accordance with various embodiments of the present invention.
FIG. 4 depicts a block diagram of an electrosurgical generator in accordance with various embodiments of the present invention.
FIG. 5 depicts a block diagram of an embodiment of a control system of an electrosurgical generator coupled to an electrosurgical hand device.
FIG. 6 depicts, in greater detail, a block diagram of an embodiment of a feedback system within a control system of an electrosurgical generator.
FIG. 7 is a schematic illustration of an example of hardware resources implemented within an RF amplifier of an electrosurgical generator to perform self-verification system test.
FIGS. 8-9 illustrate flow diagrams of various embodiments of a self-verification system operations or processes in accordance with various embodiments of the present invention.
FIG. 10 illustrates a flow diagram of an example method for performing a self-verification system test of an electrosurgical generator.

In the appended figures, similar components and/or features may have the same reference label. Where the reference label is used in the specification, the description is applicable to any one of the similar components having the same reference label.

### DETAILED DESCRIPTION

The ensuing description provides preferred exemplary embodiment(s) only, and is not intended to limit the scope, applicability or configuration of the disclosure. Rather, the ensuing description of the preferred exemplary embodiment(s) will provide those skilled in the art with an enabling description for implementing a preferred exemplary embodiments of the disclosure. It should be understood that various changes may be made in the function and arrangement of elements without departing from the spirit and scope of the invention as set forth in the appended claims.

This disclosure relates in general to electrosurgical systems. It specifically relates to a new generation of electrosurgical generators capable of initiating and performing an auto-verification and self-verification system test of the generators.

To maintain an electrosurgical generator, verification of the generator's output is often required to ensure the RF being outputted from the generator is as intended. This verification can be performed periodically, e.g., every twelve months or two years and often by a user interactive or manual system or process. The system or process may also require the connection of various external devices to the generator. These external devices may include a verification adapter, external loads, e.g., 10-500 Ohm loads with decreasing power ratings and increasing voltage ratings, and/or measuring devices, e.g., oscilloscope, differential voltage probe and current probe.

The user must then connect the appropriate adapter, loads and/or measuring devices and perform specified tests requiring adjustments to the generator, external devices and/or measuring devices and connections thereto. Additionally, appropriate monitoring and recording the test results are required along with understanding errors and physically removing a faulty generator from use. Also, to perform the generator's output verification, the generator must be taken out of circulation and not be used in a surgical procedure, and often times not performed at a surgical site such as, for example, hospitals, due to lack of experience, personnel or equipment.

Embodiments of the present invention are directed to systems and methods for enhancing surgical outcomes by providing generators having accurate RF energy generation, measurement, calibration and/or self-testing system. The present invention in accordance with various embodiments allows detection of failure upon activation, thereby reducing troubleshooting and surgical operational time while avoiding other potential surgical difficulties. This is achieved through implementation of an automated self-verification process at a power start-up of the electrosurgical generator, which allows for rapidly identifying a potential generator issue prior to any use of a connected electrosurgical instrument or supply of any RF energy to the tissue or vessel through the electrosurgical instrument.

The electrosurgical generators in accordance with various embodiments may include one or more internal impedance load for performing the self-verification process. The internal impedance load with multiple configurations are utilized to verify the voltage, current, power, and/or phase measurements of the generator. By incorporating or integrating the self-verification process and its related hardware resources within the electrosurgical generator, many improvements in outcome of pre-surgical procedures may be achieved. By way of examples, these improvements may include, but not limited to, elimination for connecting external devices to the generator, thereby removing the risk of using unavailable or unreliable test equipment, and/or unavailable or inexperienced personnel. In addition, the need for taking the generator out of circulation for performing the generator's output verification is completely eliminated.

In the following, the electrosurgical system and method according to various embodiments of the present invention is explained in detail with sections individually describing: the electrosurgical generator, the electrosurgical instrument and the control system and method used for performing the automated self-verification system test of the generator.

In accordance with various embodiments, an electrosurgical generator is provided that controls the delivery of electrosurgical or radiofrequency (RF) energy, adjusts the RF energy and in various embodiments measures and monitors electrical properties, e.g., phase, current, voltage and/or power, of the supplied RF energy to a connectable electrosurgical instrument to ensure optimal sealing, fusing and/or cutting of tissues or vessels. In various embodiments, the generator may include a feedback system that determines such electrical properties and through a microcontroller regulates and/or controls an RF amplifier that generates the required RF energy to provide the optimal RF output for sealing, fusing and/or cutting tissue/vessels under dynamic conditions, such as for example, varying loads, procedural or operational conditions.

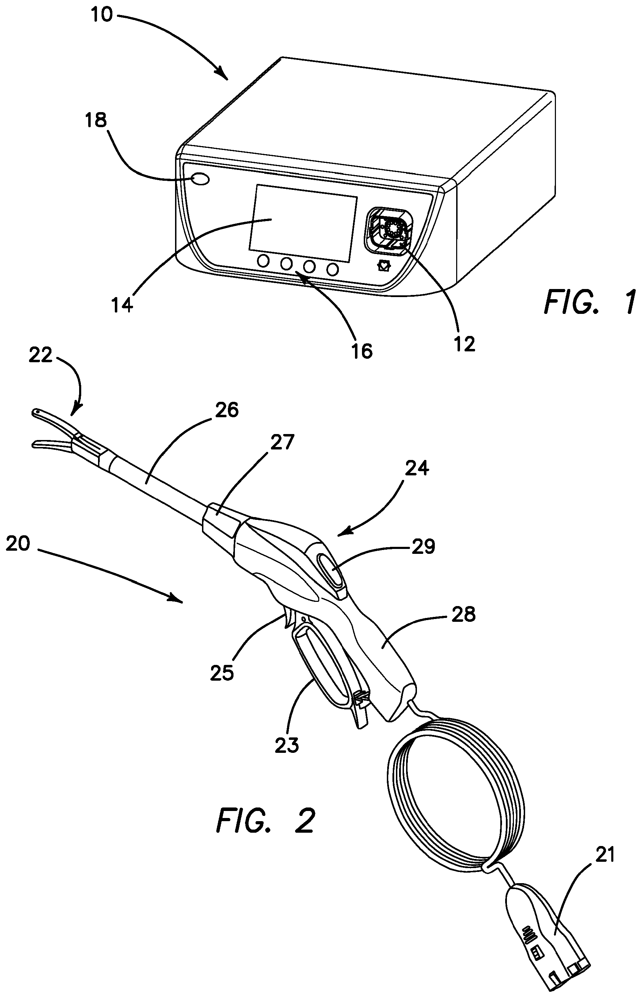

Referring first to **FIGS. 1-2**, an exemplary embodiment of an electrosurgical system according to various embodiments of the present invention is illustrated. As shown in these figures, the electrosurgical system may include an electrosurgical generator **10** and a removably connectable electrosurgical tool or instrument **20**. The electrosurgical hand device or instrument **20** can be electrically coupled to the generator **10** via a cabled connection with a device key or connector **21** extending from the instrument **20** to a device connector or access port **12** on the generator **10**. The electrosurgical instrument **20** may include audio, tactile and/or visual indicators to apprise a user of a particular or predetermined status of the instrument **20** such as, for example, a start and/or end of a fusion operation. In some embodiments, a manual controller such as a hand or foot switch can be connectable to the generator **10** and/or instrument **20** to allow predetermined selective control of the instrument such as to commence a fusion operation.

In accordance with various embodiments, the electrosurgical generator **10** includes a display **14** that may indicate the status of the electrosurgical system including, among other information, the status of the one or more electrosurgical instruments and/or accessories, connectors or connections thereto, the state or operations of the generator and error indicators. The electrosurgical generator **10** in accordance with various embodiments of the present invention may include a user interface such as, for example, a plurality of buttons **16**. The plurality of buttons **16** allows for user interaction with the electrosurgical generator **10**. This user interaction may include, for example, requesting an increase or decrease in the electrical energy supplied to one or more instruments **20** that are coupled to the electrosurgical generator **10**. In various embodiments, the generator **10** further includes a user-accessible power-on switch or button **18** that when activated powers the generator **10** and activates or initiates a self-verification system test of the generator. In other embodiments, the display **14** can be a touch screen display thus integrating data display and user interface functionalities.

In various embodiments, the electrosurgical generator **10** of the present invention is configured to output radiofrequency (RF) energy through the connectable electrosurgical instrument or hand device **20** to seal, fuse and/or cut tissue or vessels via one or more electrodes. The electrosurgical generator **10**, according to the embodiments of the present invention, is configured to generate up to 300V, 8A, and 375VA of RF energy and it is also configured to determine a phase angle or difference between RF output voltage and RF output current of the generator during activation or supply of RF energy. In this way, the electrosurgical generator **10** regulates voltage, current and/or power and monitors RF energy output (e.g., voltage, current, power and/or phase). In one embodiment, the generator **10** may stop, terminate or otherwise disrupt RF energy output under predetermined conditions. By way of example, these predetermined conditions may be any of the following conditions: when a device switch is de-asserted (e.g., fuse button released), a time value is met, and/or active phase angle and/or change of phase is greater than or equal to a phase and/or change of phase stop value indicating end of an operation such as fusion or cutting of tissue.

The electrosurgical instrument **20**, according to the embodiments of the present invention, may include an elongate shaft **26** having a proximal end coupled to or from which an actuator **24** extends and a distal end coupled to or from which jaws **22** extend. A longitudinal axis extending from the proximal end to the distal end of the elongate shaft **26**. In one embodiment, the actuator **24** may include a movable handle **23** which is pivotably coupled to a stationary handle or housing **28**. The movable handle **23** is coupled to the stationary handle or housing **28** through a central or main floating pivot. In operation, the movable handle **23** is manipulated by a user, e.g., a surgeon, to actuate the jaws **22** at the distal end of the elongate shaft **26**, and thereby, selectively opening and closing the jaws **22**. When tissue or vessels are grasped between the jaws **22**, a switch or button **29** is activated by the surgeon to seal, fuse and/or cut the tissue/vessels between the jaws **22**. Once the button **29** is activated, associated circuitry or contacts are connected to connect appropriate electrodes of the jaws with associated connections of the generator **10** to supply RF energy to tissue grasped between the jaws **22** or otherwise in contact with the one or more electrodes of the jaws.

In various embodiments, the electrosurgical instrument **20** further includes a mechanical or electrical cutting blade that can be coupled to a blade actuator such as a blade lever or trigger **25** of the stationary handle or housing **28**. The cutting blade is actuated by the blade trigger **25** to divide or cut the tissue between the jaws **22**. In various embodiments, a blade slider is connected to the blade trigger **25** and a protrusion extends from a proximal portion of the blade slider into an opening in one end of the blade trigger connecting the components together. The other end of the blade trigger is exposed and accessible by the user with the blade trigger **25** being pivotable about a trigger pivot at or near the midpoint of the blade trigger. As such, as the blade trigger **25** is pulled or rotated by the user proximally, the end of the blade trigger connected to the blade slider slides or moves the blade slider distally. Integrated with or attached to a distal end of the blade slider is a cutting blade, knife or cutting edge or surface. As such, as the blade slider translates longitudinally through a blade channel in the jaws, tissue grasped between the jaws **22** is cut. In one embodiment, the cutting edge or surface is angled to facilitate cutting of the tissue between the jaws **22**. In various embodiments, the cutting blade is a curved blade, a hook, a knife, or other cutting element that is sized and configured to cut tissue between the jaws **22**.

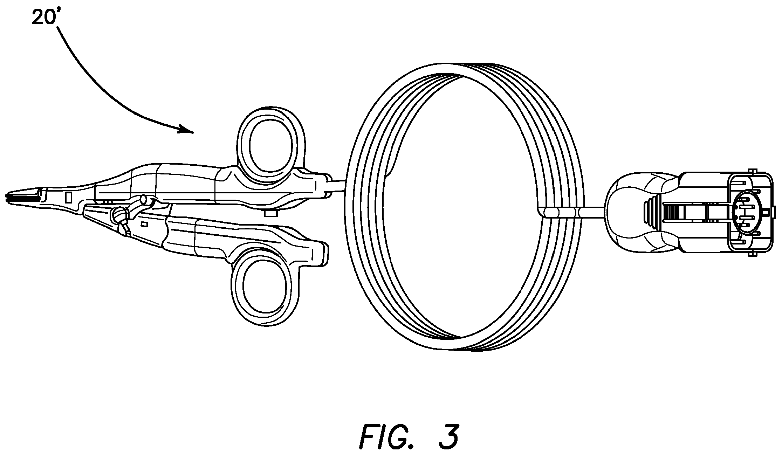

In accordance with various embodiments, the elongate shaft **26** comprises an actuation tube or rod coupling the jaws **22** with the actuator. In one embodiment, the actuator includes a rotation shaft assembly including a rotation knob **27** which is disposed on an outer cover tube of the elongate shaft **26**. The rotation knob **27** allows a surgeon to rotate the shaft of the device while gripping the actuator. In various embodiments, the elongate shaft **26** is rotatable 360 degrees and in other embodiments, rotation of the elongate shaft **26** is limited to 180 degrees, i.e., ninety degrees clockwise and ninety degrees counter clockwise. **FIG. 3** illustrates an alternative embodiment of an electrosurgical hand device **20'** connectable to the electrosurgical generator **10**. The electrosurgical hand device **20'** is similar but includes different features and has a different surgical use than the electrosurgical hand device **20**.

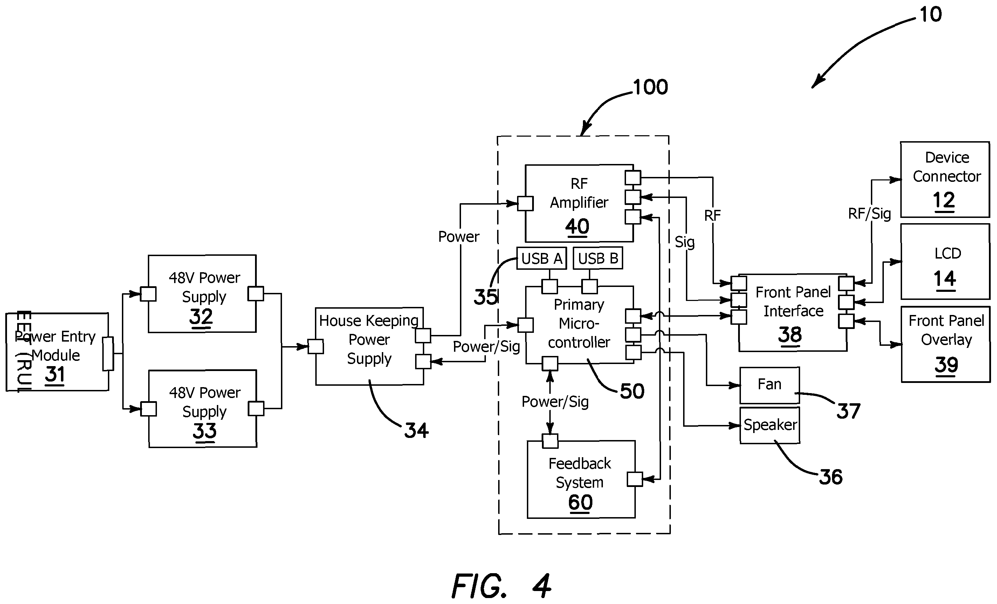

Referring next to **FIG. 4**, a block diagram of an electrosurgical generator **10** according to the embodiments of the present invention is shown. As shown in this figure, the electrosurgical generator **10** may include a power entry module **31**, e.g., an AC main input, coupled to a power supply module, e.g., two 48V DC power supplies **32, 33**. The power supply module converts the AC voltage from the AC main input to a DC voltage and via a house keeping power supply **34** provides power to various circuitry of the generator **10** and in particular supplies power to an RF amplifier **40** that generates or outputs the RF energy. In one embodiment, the RF amplifier **40** may include a combined Buck and H-Bridge circuitry to convert a DC voltage input into an RF output and in another embodiment into a variable amplitude 350kHz sine wave. The DC voltage input is a 96V DC input that is generated by the two 48V DC power supplies **32, 33** coupled in series. One of the 48V DC power supply **32, 33** is configured to generate low voltage rails and in particular supply standby voltage to power on the generator **10**.

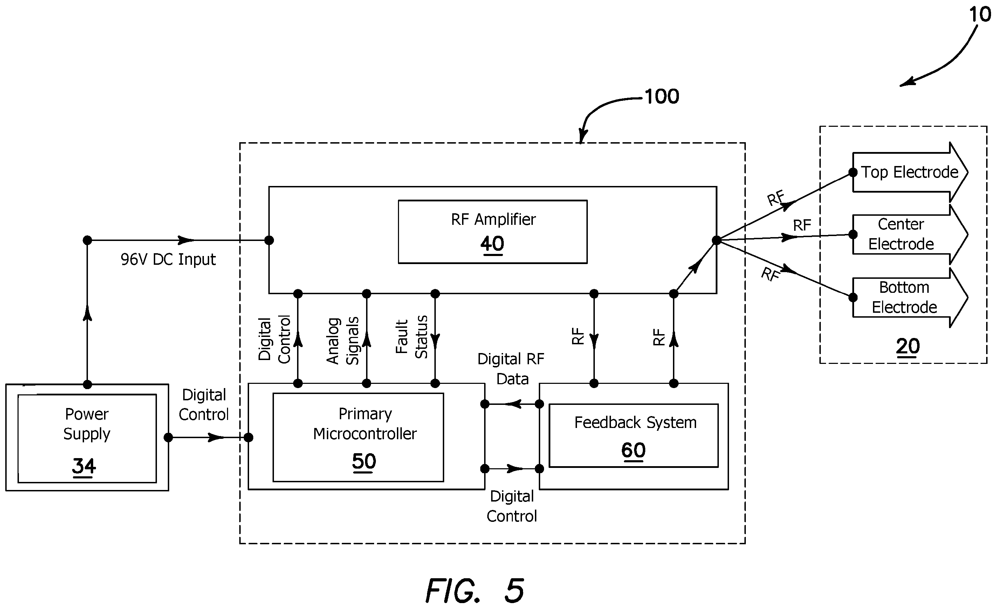

The RF output and in various embodiments the amplitude of the RF waveform output is controlled and regulated by an electrosurgical control system or a digital integral servo control system **100** embedded or integrated within the electrosurgical generator **10**. **FIG. 5** illustrates, in greater detail, a block diagram of an embodiment of a control system **100** of the electrosurgical generator **10** coupled to an electrosurgical hand device **20**. As shown in FIGS. 4-5, the control system **100** may include the RF Amplifier **40**, a primary microcontroller **50** and a feedback system **60**. The control system **100** varies between regulating voltage, current, or power of the RF output generated by the RF Amplifier **40**. In various embodiments, the feedback system **60** measures the RF output and, after processing the measured data, digitally feeds the RF output's real and imaginary components to the primary microcontroller **50**. The primary microcontroller **50**, according to the embodiments of the present invention, may include a primary field programmable gate array (FPGA) and a processor. By way of example, the processor of the microcontroller **50** may include an advanced reduced instruction set machine (ARM) processor. The primary FPGA processes the received data from the feedback system **60** and adjusts the output of the RF amplifier **40** to meet a desired regulation target. In various embodiments, the feedback system **60** comprises of analog input, digital processing and digital output.

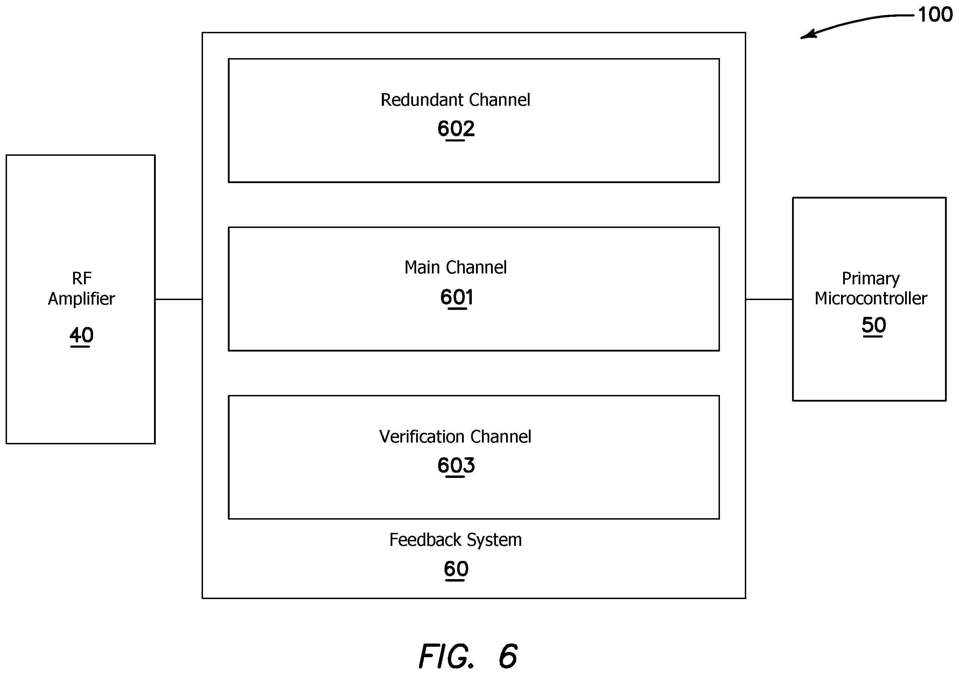

With reference to **FIG. 6**, a block diagram of an embodiment of the feedback system **60** within the control system **100** of the electrosurgical generator **10** is shown in greater detail. As shown in this figure and in accordance with various embodiments of the present invention, the verification system **60** may include a main channel **601** and a redundant channel **602**. The main channel **601** and redundant channel **602** in various embodiments may include separate but identical components. Additionally, the main and redundant channels **601** and **602** follow separate but identical electrical paths and in one embodiment are both connected to the RF amplifier **40** and the RF output.

The feedback system **60** further includes a verification channel **603** which in various embodiments is separate but similar to the main channel **601** and redundant channel **602**. The verification channel **603** may include components that are separate from the other channels but are similar. In one embodiment, the verification channel **603** may include the same components as the main and redundant channels **601** and **602**, but the components in the verification channel **603** have higher ratings, e.g., higher resolution and/or lower drift, and are often more costly. In another embodiment, the verification channel **603** may include the same components as the main and redundant channels **601** and **602**. However, in various embodiments, the verification channel **603** is only used in the operation of or by the self-verification test system. As such, the components of the verification channel **603** are used less often and thus may remain more accurate, e.g., have less drift, than the components of the main and/or redundant channels **601** and **602** that are constantly used throughout the generator's operations. In various embodiments, the verification channel **603** follows a separate but identical electrical path as the main and redundant channels **601** and **602** and in one embodiment is connected to the RF amplifier **40** and the RF output.

The channels 601, 602 and/or 603 in accordance with various embodiments comprises circuitry including active and/or passive components and electrical pathways connected thereto arranged to transmit and/or measure the RF energy supplied from the RF amplifier to the microcontroller and/or as directed by the microcontroller. In accordance with various embodiments, one or more or all the channels includes and/or are connected to one or more measurement circuitry comprising sensors, detectors, comparators, resistors, memory and/or the like and/or any combination thereof configured to measure, calculate and/or record such measurements of electrical properties (e.g., current, voltage, power, and/or phase) of the RF energy directed across the one or more or all the channels.

In accordance with various embodiments of the present invention, the electrosurgical generator **10** is further configured to provide RF output in three resolution settings: low voltage, normal or medium voltage and high voltage ranges. In various embodiments, device scripts stored and located on connectable electrosurgical hand devices, e.g., instrument **20**, and/or connectors coupled thereto, e.g., device key **21**, are used to determine or set the RF output or voltage mode.

In various embodiments, the electrosurgical generator **10** logs all RF output data onto an internal memory device, e.g., a secure digital (SD) or non-volatile memory card. The memory device is configured to be read through an interface port **35**, e.g., a universal serial bus (USB) port, on the electrosurgical generator **10** (best shown in FIG. 4). In various embodiments, the generator **10** is configured to copy the data from the internal memory device to a connectable portable storage device, e.g., a USB flash drive, through the interface port of the generator.

Reffering back to FIGS. 1 & 4-5 and in accordance with various embodiments, the electrosurgical generator **10** is configured to alert the surgeon when the vessel has reached a completed procedure state, e.g., a completed seal state, or if an error or fault condition has occurred. The electrosurgical generator **10** in various embodiments may include visual, tactile and/or audible outputs to provide such alerts or other indicators or information to the surgeon as dictated by the surgical procedure, device script or health or operational information regarding the device **20** and/or generator **10**. In one embodiment, the generator **10** via a front panel interface **38** alerts the surgeon through the LCD display **14**, which is integrated into a front panel of the generator, and in various embodiments provides specific audible alarm or informational tones through a speaker **36** also integrated into the front panel of the generator. The generator **10** in various embodiments may include a front panel overlay **39** that provides a user interface or access including navigational push buttons to allow user access to systems settings such as volume or display brightness. The front panel overlay **39** may also include the system power button or connection. In various embodiments, a fan system **37** is provided to assist in heat dissipation. Additionally, as illustrated in the FIGS. 4-5, signal or sig represents connections that, for example, comprise of digital signals used to communicate information across systems and/or printed circuit boards, power represents connections that, for example, comprise of voltage rails used to power systems and/or printed circuit boards and RF represents connections that, for example, comprise of high voltage, high current RF energy used to seal, fuse or cut tissue or vessels.

As described further above, the electrosurgical generator **10** in various embodiments further includes the user-accessible power-on switch or button **18** that is accessible by a surgeon to activate or turn on the generator **10**. In one embodiment, the power-on switch **18** is on a front panel of the generator. In accordance with various embodiments, once the generator **10** is activated by the activation of the power-on switch **18**, the generator **10** initiates or activates a power on self-verification system test. During the self-verification system test, in various embodiments, the generator **10** verifies regulation of the RF output in one or more RF modes and/or one or more RF resolution settings. In accordance with various embodiments, the RF regulation modes include voltage, current and power regulation modes and the RF resolution settings include low, normal and high voltage settings.

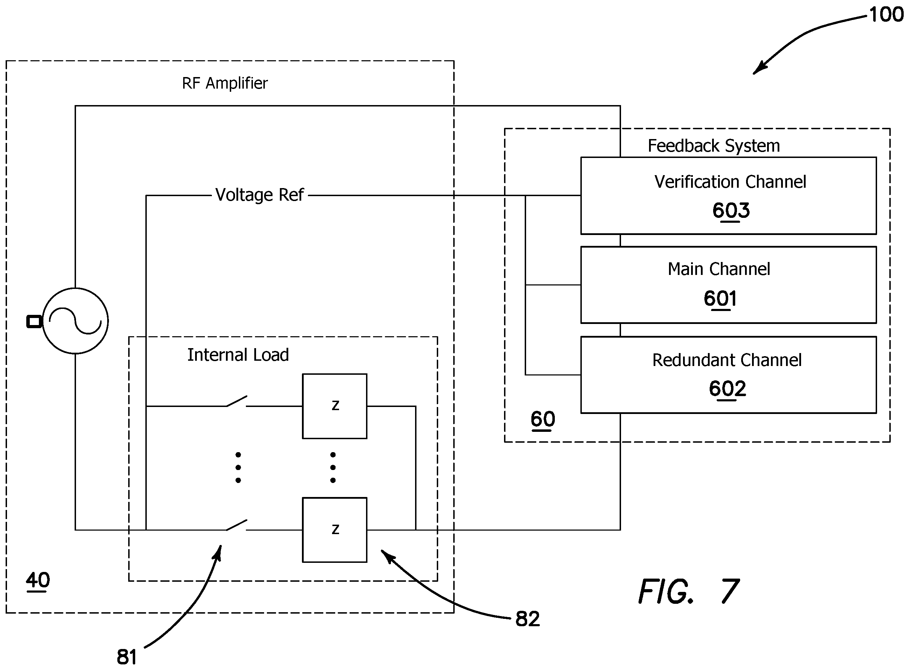

With reference to **FIG. 7**, a schematic illustration of an example of hardware resources implemented within an electrosurgical generator **10** to perform self-verification system test is shown. As it can be seen from this figure, one or more impedance load **82** may be implemented within the RF amplifier **40** of the control system **100**. In various embodiments, the control system **100** from the electrosurgical generator **10** may include one or more impedance loads **82** which are internal and/or integrated within the generator **10**. It should be understood that the impedance loads **82** are not user-accessible and in various embodiments they are only used for the purpose of performing the self-verification system test of the generator **10**. The one or more impedance loads **82**, according to the embodiments of the present invention, may be resistive, capacitive, inductive, and any combination thereof. The self- verification system test process utilizes the impedance loads **82** to verify the voltage, current, power, and/or phase measurements of the generator **10**.

In some embodiments, the impedance loads **82** are attached or integrated into the RF amplifier **40**. In other embodiments, various configuration of the impedance loads **82** may be selected via one or more relays or switches **81**. In one embodiment, the impedance loads **82** are in a parallel configuration. In another embodiment, the impedance loads **82** are in series, parallel or a combination thereof to provide different load configuration or values for other self-verification settings or testing.

The impedance loads **82**, according to the embodiments of the present invention, are internal to avoid potential inaccuracies or errors due to the use of external impedance loads, such as connections, e.g., variable losses due to cabling, load properties, e.g., phase changes, user error, and equipment tolerances or errors thereto. The internal or integrated self-verification system including but not limited to the internal impedance loads **82** also avoids the need for additional measurement equipment, e.g., oscilloscopes, or specialized equipment, e.g., test electrosurgical instruments or keys, or accessories, e.g., adapters, along with any potential inaccuracies associated with the use of such equipment. Also, any setup time or scheduling of time to perform such verification is also avoided through the automatically scheduled and executed self-verification system test.

T**able I** summarizes exemplary self-verification processes performed in various RF regulation modes and RF resolution settings. In accordance with various embodiments, each process step may have specific regulation values and impedance loads configuration. After setting the regulation values and activating the verification relays **81**, the RF amplifier **40** generates the RF output and supplies the RF output to the internal impedance loads **82** implemented within the RF amplifier **40** as directed by the self-verification system test. Accordingly, the control system **100** regulates the RF output to the set value and the feedback system **60** measures various electrical characteristics of RF output from the main channel **601**, redundant channel **602** and verification channel **603** and feeds digitally the measured results to the microcontroller **50** for further processing. The primary microcontroller **50** compares the measured data or readings of the main and redundant channels 601 and 602 to the verification channel 603 and accordingly initiates or halts the supply of RF energy.

| **Test** | **Voltage Mode** | **Regulation Unit** | **Regulation Value** | **Load** |
|---|---|---|---|---|
| **1** | **High** | **Voltage** | **~200V** | **Open** |
| **2** | **Normal** | **Voltage** | **~80V** | **Open** |
| **3** | **Normal** | **Voltage** | **~60V** | **Open** |
| **4** | **Normal** | **Voltage** | **~60V** | **Resistor & Capacitor** |
| **5** | **Normal** | **Current** | **~1A** | **Resistor & Capacitor** |
| **6** | **Normal** | **Power** | **~80W** | **Resistor & Capacitor** |
| **7** | **Normal** | **Voltage** | **~40V** | **Resistor** |
| **8** | **Normal** | **Current** | **~1A** | **Resistor** |
| **9** | **Normal** | **Power** | **~80W** | **Resistor** |
| **10** | **Low** | **Passive - Limited** | **N/A** | **Resistor** |

In accordance with various embodiments, in order for the self-verification tests to pass, the measurements of all the feedback system channels **601, 602** and **603** must match each other and/or be within a certain tolerance and/or match a target RF set point. More specifically, the main channel **601,** redundant channel **602,** and verification channel **603** have to be within a certain tolerance of a nominal value based on the internal load impedance configuration and target RF set point to prevent false positives if all readings of three channels are identical but offset. For example, if all the feedback system channels **601, 602** and **603** read or measure 20 volts from the RF output on a 60 volt test, the test will fail or not pass even though the channels identified identical voltage measurements.

In accordance with various embodiments, at the factory level after an electrosurgical generator **10** passes top level calibration and verification in the production process, initial verification channel offsets are calculated. The initial offset values are calculated by comparing the main and redundant channel readings to that of verification channel and offsetting them to the verification values. As a result, this sets the main, redundant, and verification channel readings to identical values. The primary microcontroller **50,** in various embodiments, utilizing the ARM processor, applies these initial offsets internally for each verification test or process. Therefore, for example, if the internal impedance loads, e.g., shunt resistors, on any of the main, redundant and verification channels **601, 602** and **603** start to drift after the initial offset adjustment the self-verification system test of the generator **10** will identify the drift and/or otherwise indicate an error or failure.

A self-verification system test failure, according to the embodiments of the present invention, will display on the generator's LCD **14** and/or the generator **10** will not function such that no RF energy will be supplied to a connected electrosurgical instrument **20**. In various embodiments, the generator's power can be cycled off and on and the self-verification system test will initiate again and, in various embodiments, after a predetermined number of failures in a particular sequence, e.g., two verification failures in a row, the electrosurgical generator **10** will fault or enter into a nonfunctional state and will require service before being able to operate, e.g., be able to supply RF energy to a connected electrosurgical instrument **20**.

With the self-verification system operating at the generator or system power-on or startup, the electrosurgical generator **10** is configured to notify the surgeon of a potential generator issue, prior to any use of a connected electrosurgical instrument **20** or any supply of RF energy to the tissue or vessel through the electrosurgical instrument **20**.

As such, without the self-verification system during the generator's startup or power-on operation, a conventional generator would not detect an RF output failure until a surgeon attempted to apply RF energy to tissue, if at all. The electrosurgical generator **10** according to the embodiments of the present invention is thus configured to detect failures on startup or a predetermined schedule which reduces troubleshooting and surgical operational time and avoids other potential surgical difficulties.

In various embodiments, the verification channel **603** has components of lower drift and higher resolution (higher quality and lower PPM/°C). As such, the low thermal coefficients, which can dictate the drifts of the components, ensures that the verification channel **603** is more resistant to change over time and temperature. Additionally, components, such as shunt resistors, of the verification system channels **601, 602** and **603** may drift in value over time due to heat generated by the RF output. This drift can skew measurement values. However, since the components, e.g., the impedance loads, of the verification channel **603** are not utilized during normal RF output operations, the verification channel **603** is more resistant to drift.

As described further above, the verification channel **603** uses the normal RF output path, which is then redirected using relays **81** to self-verification resistors and capacitors, e.g., impedance loads **82**, inside the electrosurgical generator **10** instead of the normal RF output path to a connected electrosurgical hand device **20**. In various embodiments, high power loads, e.g., resistors, inductors or capacitors, are used to withstand or resist adverse effects, such as temperature, introduced through the application of RF energy. In one embodiment, high power resistors are provided and in other embodiments there are chassis mounted and placed in the airflow path of the generator to enhance heat dissipation.

In some embodiments, the impedance loads of the verification channel **603** are included or integrated on the same circuit board as the other channels **601** and **602** of the feedback system **60** that reduces the overall footprint of the electrosurgical generator **10** or the feedback system **60**. In other embodiments, the impedance loads of the verification channel **603** are included or integrated on the different circuit board thereby reducing any affect to surrounding components due to heat generated by the impedance loads. In various embodiments, the impedance loads are varied, e.g., different value components, e.g., resistors, or different components, e.g., inductors used instead of capacitors for phase measurements, to enhance additional variations in the regulation modes being verified. In addition, the impedance loads may be in series, parallel or any combination thereof to provide various load configuration or values.

In some embodiments, the verification channel measurement circuit is positioned in series with the RF output circuit enhancing accuracy of the measurements and easing tracking or recording of such measurements. In other embodiments, the verification channel measurement circuit is separated, e.g., via a relay, from the RF output circuit and thus can enhance the resistant of the verification channel measurement circuit due to reduced operation time or the effects of heat due to supply of RF energy.

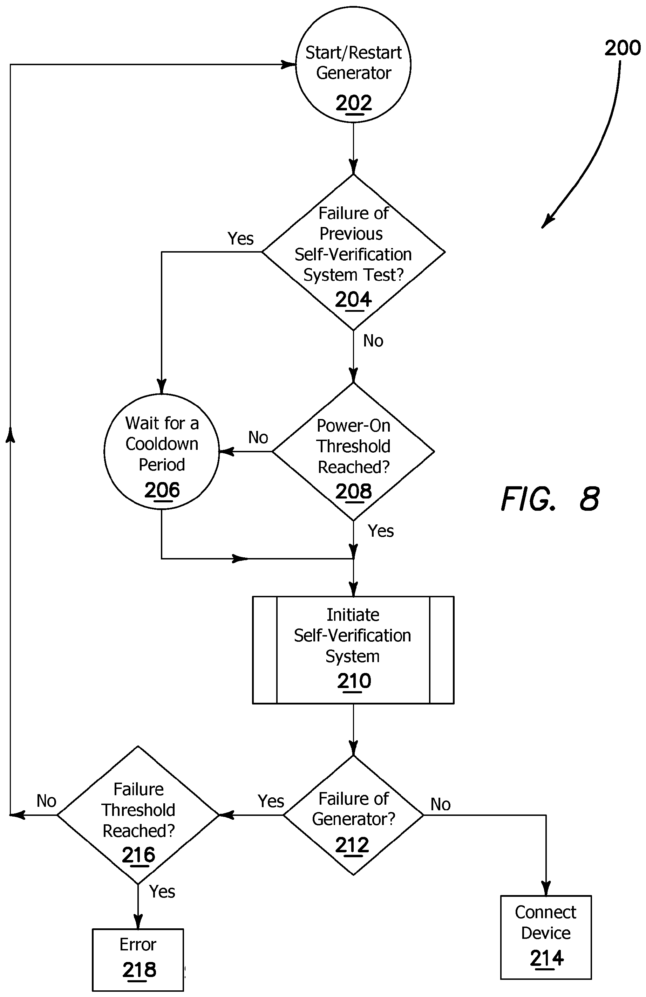

Referring next to **FIG. 8**, an embodiment of a self-verification system operations or processes according to the embodiments of the invention is shown. The depicted portion of the process **200** begins in step **202** where the algorithm powers on the electrosurgical generator **10** as a starting point. After starting-up the generator **10**, a determination is made, at step **204**, as to whether the previous self-verification system test has failed. If failure of the previous self-verification system test is detected, processing flows from block **204** to block **206** where the processing waits for a predetermined period of time (cooldown period) or power-on threshold, e.g., one minute, prior to initiating the self-verification system test at block **210**. If failure of the previous self-verification system test is not detected, processing goes from block **204** to block **208** where another determination is made as to whether the predetermined period of time or power-on threshold has elapsed after the electrosurgical generator **10** is started.

If the power-on threshold is reached or exceeded, processing flows from block **208** to block **210** to initiate the self-verification system test. If the power-on threshold is not reached in block **208**, processing goes to block **206** to wait for the predetrmined period of time (cooldown period) or power-on threshold to pass. The processing then goes to block **210** for initiating the self-verification system test.

Once the self-verification system test is initiated at block **210**, the electrosurgical generator **10** is verified using various processing tests. After completion of the self-verification system test, processing continues to block **212** where a determination is made as to whether a failure of the electrosurgical generator **10** has been detected during the self-verification process. If a failure is not detected, processing flows from block **212** to block **214** where the electrosurgical generator **10** is configured to supply RF energy to a connected electrosurgical hand device **20**. In this embodiment, the electrosurgical generator **10** may provide a display or other indication that an electrosurgical device **20** can be connected to the generator **10** or the device already connected to the generator **10** is ready for surgical use. In other embodiments, device authentication and/or verification is also performed by the electrosurgical generator **10**, prior to and/or after the generator **10** is verified by the self-verification system, before the connected electrosurgical instrument **20** is ready for surgical use. In accordance with various embodiments of the present invention, the processing determines that the electrosurgical generator **10** has no failure if one or more or all of the verification processes or tests are passed successfully.

If a failure of the electrosurgical generator **10** is detected, processing goes from block **212** to block **216** where another determination is made as to whether a failure threshold has been reached. The failure threshold determines if the self-verification system has failed consecutively a predetermined number of times, e.g., twice or more in a row. If the failure threshold is reached, processing flows from block **216** to block **218** where an error is generated causing the generator **10** to be inoperable. In this embodiment, the inoperative state of the generator **10** requires that the electrosurgical generator **10** to be serviced. In accordance with various embodiments of the present invention, the electrosurgical generator **10** notifies the user or surgeon, of the generator's error via an audible, tactile and/or visual indicator.

If the failure threshold is not reached in block **216**, the processing then goes back to block **202** for restarting the electrosurgical generator **10** in which the processing can again attempt to verify the generator **10**. This process continues until a failure of the electrosurgical generator **10** is not detected or the failure threshold is reached or exceeded.

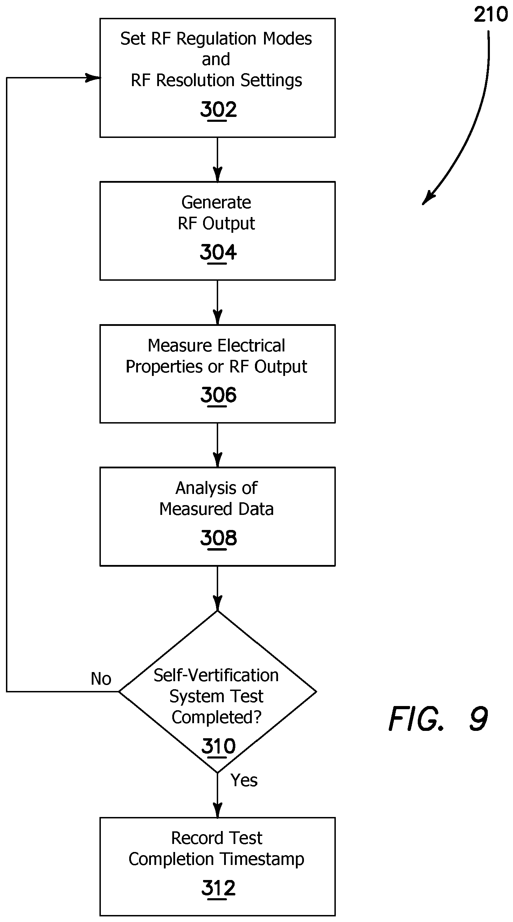

With reference to **FIG. 9**, a flow diagram of an embodiment of process **210** for performing self-verification system test is shown. Self-verification process steps for various RF regulation modes and RF resolution settings are typically recorded or stored into a memory of the electrosurgical generator **10** of the present invention. In accordance with various embodiments, each process step may have specific regulation values and impedance loads configuration. Exemplary self-verification processes were provided further above in Table I. After initiating the self-verification system test, the depicted portion of the process begins in blocks **302** where the algorithm sets the RF regulation modes and RF resolution settings. As such, the processing sets the voltage mode, activates appropriate relays **81** for obtaining specific load configuration and sets the regulation value. The processing then goes to block **304** for generating RF output and providing RF energy to the verification system loads. In various embodiments, the RF amplifier **40** generates the RF output and supplies the RF output to internal loads **82** within the amplifier as directed by the self-verification system.

Once the RF output is generated, processing flows to block **306** where the feedback system **60** measures the electrical characteristics of the RF output. The control system **100**, in accordance with various embodiments of the present invention, regulates the RF output to the set value as directed by the self-verification system and the feedback system **60** measures voltage, current, power, and/or phase from the main, redundant and verification channels **601**, **602**, and **603**. After measuring the electrical characteristics of the RF output, processing flows to block **308** where the primary microcontroller **50** performs calculations, comparison and analysis of the measured data. In various embodiments, the feedback system **60** communicates the measured data and/or real and imaginary components thereof for the channels **601**, **602**, and **603** to the primary microcontroller **50** for further processing. The primary microcontroller **50** in various embodiments compares the data or readings of the main and redundant channels **601** and **602** to the verification channel **603** and if the main and redundant readings are less than a predefined tolerance or difference, e.g., 5%, the self-verification process step or test is passed or verified. Otherwise, the verification process step or test fails. In accordance with various embodiments of the present invention in both cases the results are recorded or stored into a memory of the electrosurgical generator **10** and/or the device key **21** of the electrosurgical instrument **20**.

A determination is made at block **310** as to whether the self-verification system test of the electrosurgical generator **10** has been completed. If the self-verification system test is completed, the processing flows from block **310** to block **312** where a timestamp for recording test completion is generated before exiting the self-verification process. If the self-verification system test is not completed, the processing then goes back to block **302** for setting the new RF regulation mode and new RF resolution setting. This process continues until all of the self-verification process steps for verifying the electrosurgical generator **10** are performed. In various embodiments, the self-verification system test activates at a predetermined condition, such as on power-on, device connection and/or script or device activation, and/or along a predetermined schedule, e.g., at each predetermined conditions and/or a selected interval. In various embodiments, a separate button, switch or the like is provided on the generator, a connectable device and/or adapter utilized to activate the self-verification test.

The electrosurgical generator **10**, according to the embodiments of the present invention, is configured to operate in one or more specific voltage modes. In various embodiments, the voltage modes are low, medium and high and in various embodiments the voltage mode adjusts or effects the feedback system **60**. Voltage mode, in various embodiments, determines gain settings on the ADCs (Digital to Analog Convertors) used in the feedback system **60** and in various embodiments, high voltage is a maximum 300V gain settings, medium voltage is a maximum 150V gain settings, and low voltage is a maximum 10V gain settings. By having different gain settings for the different voltage modes, the resolution of the measurement of the feedback system **60** is increased.

According to the embodiments of the present invention, the RF amplifier may include an autotransformer directly connected to a primary transformer of the RF amplifer to supply high voltage mode. In various embodiments, when the RF amplifier **40** is operating in the high voltage mode (autotransformer on), the RF output may reach up to 300V and 4A. On the other hand, when the autotransformer is off, the RF amplifier **40** may operate in the normal voltage mode, RF output limited to 150V and 8A, and in the low voltage mode or passive mode which is limited to 10V and 100mA.

In some embodiments, the self-verification system test varies the voltage modes to test or ensure the different gain settings are accurate across the main, redundant and verification channels **601**, **602**, and **603** of the feedback system **60**. In other embodiments, different regulation modes of voltage, current and power are also included with the various voltage modes which are utilized and tested in the self-verification system test.

In accordance with various embodiments of the present invention, the measurements and calculations provided by the main and redundant channel **601** and **602** are compared to the measurements and calculations of the verification channel **603**. If the channels **601, 602,** and **603** all match and in various embodiments within a set tolerance, then the self-verification system test according to the embodiments of the present invention clears or allows the electrosurgical generator **10** to continue and thus allow RF output to a connected electrosurgical device **20**. The main, redundant and verification channels **601, 602,** and **603** in various embodiments must also be within a set tolerance compared to a nominal regulation value to also allow the verification system to clear the generator for operation.

According to the embodiments of the present invention, the low voltage mode is only used for passive measurement and does not set a constant voltage, current, or power. In various embodiments, it also does not utilize the verification channel **603** and thus RF output is provided in low voltage. In this embodiment, voltage and current are measured, the resistance is calculated and some or all of the measurements and calculations are compared to predefined values and/or within a predefined tolerance to further clear or allow the electrosurgical generator **10** for further operations.

In various embodiments, the self-verification system determines or checks the drift of the sense devices such as, for example, resistors, capacitors or inductors of the main and redundant channels **601** and **602** which are used to measure or calculate electrical properties of the RF output. If the drift of these components is minimal or within a predefined range and/or the unit can regulate to set values appropriately, then the RF output of the electrosurgical generator **10** is determined to have no failure by the self-verification system test and the self-verification process allows the generator **10** for further operational or surgical use.

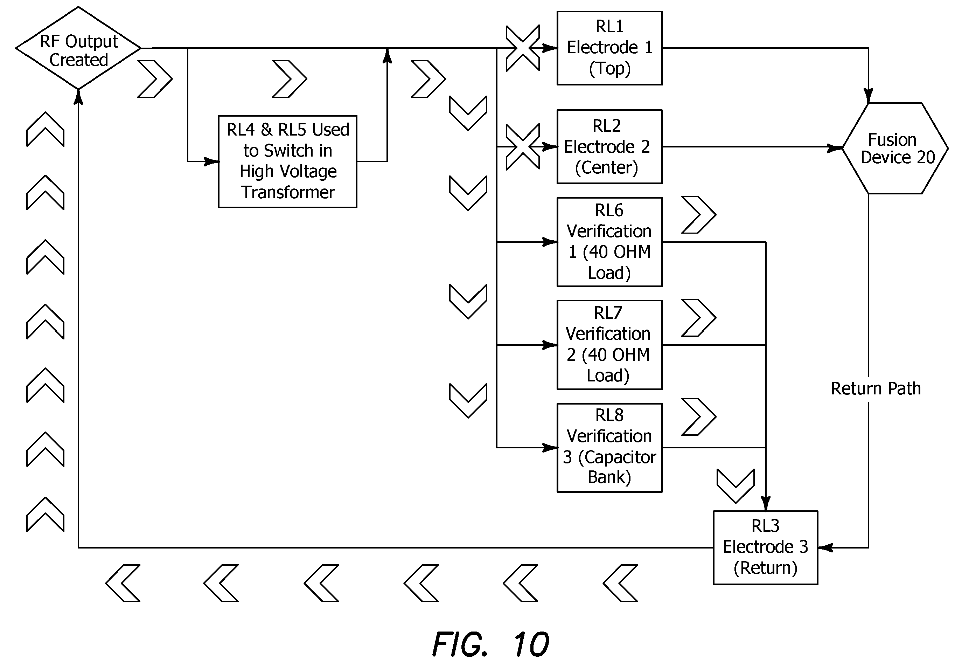

**FIG. 10** illustrates a flow diagram of an example method for performing a self-verification system test of an electrosurgical generator **10**. This process or system test verifies that the electrosurgical generator **10** can output RF energy at a set value, and that the feedback system **60** is accurately measuring this RF output across all channels **601, 602** and **603**. In accordance with various embodiments of the present invention, RF output is first generated and thus provided to the self-verification system. In various embodiments, when the generator is powered on, e.g., the power button **18** on the front panel of the generator **10** is pressed, the generator **10** initiates the self-verification system process. This will cause the RF output to be supplied or be directed to an internal load rather than through a connected electrosurgical device **20**. As shown in FIG. 10, the self-verification process bypasses or switches off relays 1 and 2 that would direct the RF output or the electrosurgical energy to a connected electrosurgical device, e.g., fusion device **20**. It should be understood that an electrosurgical device does not need to be connected to the electrosurgical generator **10** for the self-verification system to operate or to its processes to proceed. Accordingly, the processing switches toward the internal loads as directed by the self-verification system.

As illustrated in FIG. 10, relays **6-8** are switched on/off in various sequences to provide the associated internal load configuration as needed. In various embodiments, relays **6** and **7** may be activated or used simultaneously and in parallel to assist in dissipating power and/or heat. On the other hand, relay **8** in various embodiments is switched on or used to include phase variations as desired. When a higher voltage RF output is needed, relays **4** and **5** of the exemplary embodiment are used to switch in a high voltage transformer. Relay **3**, in various embodiments, is used or activated to provide the return path or complete the circuit for the self-verification system. In accordance with various embodiments of the present invention, the self-verification system may include a plurality of relays and loads including, for example, resistors, capacitors, inductors and various combinations thereof and as such other relays, loads and combinations thereof are not shown for ease of readability. In some embodiments, the self-verification system may include a timer to ensure that the self-verification system test is not activated or initiated needlessly or is activated as intended. As such, if the electrosurgical generator **10** is quickly powered on and off, e.g., in less than a second, the self-verification system does not activate and in various embodiments prevents potential thermal damage to the load such as, for example, resistors. In other embodiments, the timer is configured to start at or near power-on time and reaches or passes a predefined threshold to cause or initiate activation of the self-verification system.

The above description is provided to enable any person skilled in the art to make and use the electrosurgical devices or systems and perform the methods described herein and sets forth the best modes contemplated by the inventors of carrying out their inventions. Various modifications, however, will remain apparent to those skilled in the art. It is contemplated that these modifications are within the scope of the present disclosure. Different embodiments or aspects of such embodiments may be shown in various figures and described throughout the specification. However, it should be noted that although shown or described separately each embodiment and aspects thereof may be combined with one or more of the other embodiments and aspects thereof unless expressly stated otherwise. It is merely for easing readability of the specification that each combination is not expressly set forth.

Although the present invention has been described in certain specific aspects, many additional modifications and variations would be apparent to those skilled in the art. It is therefore to be understood that the present invention may be practiced otherwise than specifically described, including various changes in the size, shape and materials, without departing from the scope and spirit of the present invention. Thus, embodiments of the present invention should be considered in all respects as illustrative and not restrictive.

Other potentially novel aspects are defined by the following clauses.
Clause 1 An electrosurgical system for performing surgical procedures comprising:
   an electrosurgical generator adapted to perform a self-verification system test upon activation of the generator, the generator comprising:
   a processor configured to:
   cause initiating a self-verification system test after determining a predetermined period of time has elapsed after starting the generator;
   cause supplying RF energy to a connected electrosurgical hand device, if a failure of the generator is not detected; and
   cause generating a system error if the failure of the generator is detected and a failure threshold is reached; and
   a plurality of impedance loads configured for performing the self-verification system test.
Clause 2 The electrosurgical system of clause 1 wherein the processor is further configured to cause restarting the generator to reinitiate the self-verification system test if the failure of the generator is detected and the failure threshold is not reached.
Clause 3 The electrosurgical system of clause 1 wherein the failure threshold comprises a number of consecutive times where the self-verification system test detects the failure of the generator.
Clause 4 The electrosurgical system of clause 1 wherein upon activation of the generator, the processor is configured to determine whether a failure of the previous self-verification system test has occurred.
Clause 5 The electrosurgical system of clause 4 wherein if the failure of the previous self-verification system test is detected, the processor cause the generator to wait for a cooldown period.
Clause 6 The electrosurgical system of clause 4 wherein if the failure of the previous self-verification system test is not detected, the processor further determines whether a power-on threshold time has elapsed after starting the generator and prior to initiating the self-verification system test.
Clause 7 The electrosurgical system of clause 1 wherein the self-verification system test comprises a verification algorithm verifying RF output of the generator across a plurality of RF regulation modes and a plurality of RF resolution settings.
Clause 8 The electrosurgical system of clause 7 wherein the plurality of RF regulation modes comprises one of voltage, current, power and/or phase regulation mode.
Clause 9 The electrosurgical system of clause 7 wherein the plurality of RF resolution settings comprises one of a low, medium or high voltage setting.
Clause 10 The electrosurgical system of clause 7 wherein the processor is further configured to regulate the RF output of the generator to a predetermined set value for each of the plurality of RF regulation modes and RF regulation settings.
Clause 11 The electrosurgical system of clause 1 wherein the generator further comprising a feedback system configured for measuring electrical properties of RF output across a plurality of channels.
Clause 12 The electrosurgical system of clause 11 wherein the processor allows for supplying RF energy to the connected electrosurgical hand device, if all measurements values of the feedback system channels are matching each other and/or are within a certain tolerance across the plurality of RF regulation modes and RF resolution settings.
Clause 13 The electrosurgical system of clause 11 wherein the feedback system comprises of analog input, digital processing and digital output.
Clause 14 The electrosurgical system of clause 1 wherein the plurality of impedance loads are internal or integrated within the generator, wherein specific configurations of the plurality of impedance loads are attainable using a plurality of internal relays.
Clause 15 A method for performing an auto-verification and self-verification system of an electrosurgical generator prior to performing surgical procedure, the method comprising:
   initiating a self-verification system test after determining a predetermined period of time has elapsed upon activation of the generator;
   setting RF regulation modes and RF resolution settings after initiating the self-verification system test;
   generating RF energy and directing RF output to a plurality of impedance loads within the generator;
   measuring electrical characteristics of the RF output and analyzing the measured data;
   determining whether the self-verification system test has been completed; and
   recording self-verification system test completion timestamp upon completion of the self-verification system test.
Clause 16 The method of clause 15 further comprising repeating the setting, generating, measuring and analyzing steps until a completion of the self-verification system test is determined.
Clause 17 The method of clause 15 further comprising the step of initiating supply of RF energy to a connected electrosurgical hand device, if a generator failure is not detected.
Clause 18 The method of clause 15 further comprising the step of generating a system error if a generator failure is detected and a failure threshold is reached.
Clause 19 The method of clause 18 wherein the failure threshold is reached when the self-verification system test detects the generator failure for a number of consecutive times.
Clause 20 The method of clause 15 wherein the measuring step and the analyzing step are performed respectively by a feedback system and a microcontroller of the generator.
Clause 21 The method of clause 20 wherein the measuring step comprises measuring the electrical properties of the RF output across a plurality of channels from the feedback system and communicating the real and imaginary components thereof for the plurality of channels to the microcontroller.
Clause 22 The method of clause 21 wherein the analyzing step comprises receiving the real and imaginary components of the measured data, performing power calculations and comparing the measurement values of the feedback system channels for determining whether a generator failure exist.
Clause 23 The method of clause 22 wherein the generator failure exist if measurements values of the feedback system channels are not matching each other and/or are not within a certain tolerance across the plurality of RF regulation modes and RF resolution settings.
Clause 24 The method of clause 22 wherein the microcontroller initiates or halts supply of RF energy to a connected electrosurgical hand device based on the comparison results.
Clause 25 The method of clause 15 further comprising the step of storing results obtained from the measuring and analyzing steps into a memory of the generator.
Clause 26 The method of clause 15 wherein the plurality of RF regulation modes comprises one of voltage, current, power and/or phase regulation mode.
Clause 27 The method of clause15 wherein the plurality of RF resolution settings comprises one of a low, medium or high voltage setting.
Clause 28 The method of clause 15 wherein the step of setting RF regulation modes and RF resolution settings further comprises setting a predetermined RF regulation value and activating appropriate relays for obtaining specific impedance load configuration.
Clause 29 A control system for use with an electrosurgical generator adapted to perform a self-verification system test upon activation of the generator, the control system comprising:
   an RF amplifier for supplying RF energy;
   a feedback system for continually monitoring electrical properties of the supplied RF energy and generating digital RF signals relating thereto; and
   a microcontroller configured to initiate the self-verification system test and to allow supplying and halting the supplied RF energy to a connected electrosurgical hand device based at least in part on results of the self-verification system test.
Clause 30 The control system of clause 29 wherein the self-verification system test comprises a verification algorithm verifying the supplied RF energy directed to one or more impedance loads across a plurality of RF regulation modes and a plurality of RF resolution settings.
Clause 31 The control system of clause 30 wherein the plurality of RF regulation modes comprises one of voltage, current, power and/or phase regulation mode, and wherein the plurality of RF resolution settings comprises one of a low, medium or high voltage setting.
Clause 32 The control system of clause30 wherein the microprocessor is configured to regulate the RF energy outputted from the RF amplifier to a predetermined set value for each of the plurality of RF regulation modes and RF regulation settings.
Clause 33 The control system of clause 29 wherein the feedback system is configured for measuring the electrical properties of RF energy supplied to one or more impedance loads across a main channel, a redundant channel and a verification channel of the feedback system and for digitally transmitting the measured data to the microcontroller.
Clause 34 The control system of clause 33 wherein the microcontroller is configured for receiving the measured data, performing power calculations related thereto and comparing the results from the main and redundant channels to that of the verification channel.
Clause 35 The control system of clause 34 wherein the microcontroller allows for supplying energy to the connected electrosurgical hand device, if the comparison results for the main and redundant channels are within a certain tolerance across a plurality of RF regulation modes and a plurality of RF resolution settings.
Clause 36 The control system of clause 34 wherein the microcontroller allows for halting energy to the connected electrosurgical hand device, if the comparison results for the main and redundant channels are outside a certain tolerance for at least one of the plurality of RF regulation modes and RF resolution settings.
Clause 37 The control system of clause 33 wherein the verification channel comprises components of lower drift and higher resolution compared to components of the main and redundant channels.
Clause 38 The control system of clause 33 wherein the main and redundant channels have separate but identical components, wherein the main and redundant channels follow separate but identical electrical paths.
Clause 39 The control system of clause 33 wherein components of the verification channel are separate but similar to components of the main and redundant channels, wherein the verification channel follows separate but identical electrical paths compared to those of main and redundant channels.
Clause 40 The control system of clause 33 wherein components of the verification channel, main channel and redundant channel are assembled or integrated on a same circuit board.
Clause 41 The control system of clause 33 wherein components of the verification channel are assembled or integrated on a separate circuit board from that of the main and redundant channels.
Clause 42 The control system of clause 30 wherein the one or more impedance loads are internal or integrated within the RF amplifier.
Clause 43 The control system of clause 30 wherein the one or more impedance loads are resistive, capacitive, inductive, or any combination thereof and are in series, parallel or any configuration thereof for providing various load configurations.
Clause 44 An electrosurgical system comprising:
   an electrosurgical generator adapted to supply electrosurgical RF energy to tissue, the generator comprising:
      one or more impedance loads integrated within the generator to provide various load configurations;
      a microprocessor programmed to regulate the RF output of the generator to a predetermined RF regulation value across a plurality of RF regulation modes and a plurality of RF resolution settings; and
      a feedback system configured to measure electrical properties of the RF output supplied to the one or more impedance loads across a plurality of channels; and
   an electrosurgical instrument comprising at least one active electrode adapted to apply the supplied electrosurgical RF energy to tissue;
   wherein the microprocessor is further programed to compare one or more or all measurements values of the feedback system channels for each of the plurality of RF regulation modes and RF resolution settings and determine whether a fault condition exist within the generator.
Clause 45 The electrosurgical system of clause 44 wherein the microprocessor is further adapted to allow supply of electrosurgical RF energy to the electrosurgical hand device if no fault condition exist within the generator.
Clause 46 The electrosurgical system of clause 45 wherein no fault condition exist within the generator if one or more or all measurement values of the feedback system channels are matching each other and/or are within a certain tolerance for each of the plurality of RF regulation modes and RF resolution settings.
Clause 47 The electrosurgical system of clause 44 wherein the fault condition exist within the generator if one or more or all measurement values of the feedback system channels are not matching each other and/or are not within a certain tolerance for at least one of the plurality of RF regulation modes and RF resolution settings.
Clause 48 The electrosurgical system of clause 44 wherein the microprocessor is further adapted to halt supply of electrosurgical RF energy to the electrosurgical hand device if the fault condition exist within the generator and a failure threshold is reached.
Clause 49 The electrosurgical system of clause 48 wherein the failure threshold is reached when the fault condition is redetected for a number of consecutive times after the generator being restarted.
Clause 50 The electrosurgical system of clause 48 wherein the microprocessor is further programmed to restart the generator if the failure threshold is not reached.
Clause 51 The electrosurgical system of clause 44 wherein the plurality of RF regulation modes comprises one of voltage, current, power and/or phase regulation mode.
Clause 52 The electrosurgical system of clause 44 wherein the plurality of RF resolution settings comprises one of a low, medium or high voltage setting.
Clause 53 The electrosurgical system of clause 44 wherein the microprocessor is configured to wait for a predetermined period of time after starting the generator and before beginning to regulate the RF output of the generator to the predetermined RF regulation value across the plurality of RF regulation modes and the plurality of RF resolution settings.
Clause 54 The electrosurgical system of clause 44 wherein for each of the plurality of RF regulation modes and the plurality of RF resolution settings, the microprocessor is further adapted for setting the predetermined RF regulation value and activating appropriate relays for obtaining specific load configuration of the one or more impedance loads.
Clause 55 The electrosurgical system of clause 44 wherein the feedback system comprises of analog input, digital processing and digital output.
Clause 56 An electrosurgical generator comprising:
   a plurality of impedance loads integrated within an RF amplifier that supplies RF energy;
   a feedback system configured for measuring the electrical properties of RF energy directed to the plurality of impedance loads across a plurality of channels; and
   a primary microcontroller configured to initiate a self-verification system test upon activation of the generator to verify the RF output of the generator across a plurality of RF regulation modes and RF resolution setting for determining whether a generator failure exist.
Clause 57 The electrosurgical generator of clause 56 wherein during the self-verification system test the primary microcontroller is configured to regulate the RF output of the generator to a predetermined set value for each of the plurality of RF regulation modes and RF regulation settings.
Clause 58 The electrosurgical generator of clause 56 wherein the plurality of RF regulation modes comprises one of voltage, current, power and/or phase regulation mode, and wherein the plurality of RF resolution settings comprises one of a low, medium or high voltage setting.
Clause 59 The electrosurgical generator of clause 56 wherein the generator failure exist if one or more or all measurements values of the feedback system channels are not matching each other and/or are not within a certain tolerance across the plurality of RF regulation modes and RF resolution settings.
Clause 60 The electrosurgical generator of clause 56 wherein the feedback system is configured for digitally transmitting the measured data to the primary microcontroller, wherein the plurality of channels of the feedback system comprises a main channel, a redundant channel and a verification channel.
Clause 61 The electrosurgical generator of clause 60 wherein the primary microcontroller is configured for receiving the measured data, performing power calculations related thereto and comparing the results from the main and redundant channels to that of the verification channel.
Clause 62 The electrosurgical generator of clause 60 wherein, after completion of the self-verification system test, the microcontroller allows for supplying RF energy to a connected electrosurgical hand device, if the comparison results for the main and redundant channels are within a certain tolerance across a plurality of RF regulation modes and a plurality of RF resolution settings.
Clause 63 The electrosurgical generator of clause 60 wherein, after completion of the self-verification system test, the microcontroller allows for halting RF energy to the connected electrosurgical hand device, if the comparison results for the main and redundant channels are outside a certain tolerance for one of the plurality of RF regulation modes and RF resolution settings.
Clause 64 The electrosurgical generator of clause 60 wherein the verification channel comprises components of lower drift and higher resolution compared to components of the main and redundant channels, wherein the verification channel is solely used during the self-verification system test.
Clause 65 The electrosurgical generator of clause 60 wherein the main and redundant channels have separate but identical components, wherein the main and redundant channels follow separate but identical electrical paths.
Clause 66 The electrosurgical generator of clause 60 wherein components of the verification channel are separate but similar to components of the main and redundant channels, wherein the verification channel follows separate but identical electrical paths compared to those of main and redundant channels.
Clause 67 The electrosurgical generator of clause 60 wherein components of the verification channel, main channel and redundant channel are assembled or integrated on a same circuit board.
Clause 68 The electrosurgical generator of clause 60 wherein components of the verification channel are assembled or integrated on a separate circuit board from that of the main and redundant channels.
Clause 69 An electrosurgical generator comprising:
   a plurality of switchable impedance loads integrated within the generator and through which RF energy is supplied there through; and
   a microcontroller configured to switch in one or more of the plurality of switchable impedance loads to verify RF output of the generator.
Clause 70 The electrosurgical generator of clause 69 further comprising a feedback system configured to measure electrical properties of the RF energy directed to the one or more of the plurality of switchable impedance loads.
Clause 71 The electrosurgical generator of clause 70 wherein the feedback system comprises a plurality of channels including circuitry through which RF energy is supplied.
Clause 72 The electrosurgical generator of clause 69 further comprising a feedback system configured to measure electrical properties of the RF energy directed to the one or more of the plurality of switchable impedance loads across the plurality of channels.
Clause 73 The electrosurgical generator of clause 70 or 72 further comprising an RF amplifier that supplies the RF energy.
Clause 74 The electrosurgical generator of clause 73 wherein the plurality of switchable impedance loads is integrated within the RF amplifier.
Clause 75 The electrosurgical generator of any one of the previous clauses wherein the plurality of switchable impedance loads comprises at least one relay and at least one of a resistor, capacitor or an inductor.
Clause 76 The electrosurgical generator of any one of the previous clauses wherein the microcontroller switches in the one or more of the plurality of switchable impedance loads based on a RF regulation mode.
Clause 77 The electrosurgical generator of any one of the previous clauses wherein the microcontroller switches in the one or more of the plurality of switchable impedance loads based on a RF resolution setting.
Clause 78 The electrosurgical generator of any one of the previous clauses wherein the microcontroller is configured to switch in the one or more of the plurality of switchable impedance loads based on an activation of a self-verification system test.
Clause 79 The electrosurgical generator of clause 78 wherein the activation of the self-verification system test is activated when power is supplied to the microcontroller.
Clause 80 The electrosurgical generator of any one of the previous clauses wherein the microcontroller is configured to switch in the one or more of the plurality of switchable impedance loads upon activation of the generator.
Clause 81 The electrosurgical generator of any one of the previous clauses further comprising a transformer configured to receive the supplied RF energy and amplifies the RF energy supplied to the one or more of plurality of switchable impedance loads.
Clause 82 The electrosurgical generator of clause 81 wherein the transformer receives the supplied RF energy based on a RF resolution setting.
Clause 83 The electrosurgical generator of clause 81 wherein the microcontroller connects to the transformer to receive the supplied RF energy based on a RF resolution setting.
Clause 84 The electrosurgical generator of clause 72 wherein the microcontroller detects a generator failure if one or more or all measurements values of the plurality of channels do not match each other and/or are not within a certain tolerance.
Clause 85 The electrosurgical generator of clause 78 wherein during the self-verification system test the microcontroller is configured to regulate the RF output of the generator to a predetermined set value for each of a plurality of RF regulation modes and/or RF regulation settings.
Clause 86 The electrosurgical generator of clause 85 wherein the plurality of RF regulation modes comprises one of voltage, current, power and/or phase regulation mode, and wherein the plurality of RF resolution settings comprises one of a low, medium or high voltage setting.
Clause 87 The electrosurgical generator of clause 86 wherein the microcontroller detects a generator failure if one or more or all measurements values of the plurality of channels do not match each other and/or are not within a certain tolerance across the plurality of RF regulation modes and RF resolution settings.
Clause 88 The electrosurgical generator of clause 87 wherein the microcontroller prevents the supply of the RF energy to a connected electrosurgical hand device when a generator failure is detected.
Clause 89 The electrosurgical generator of clause 88 wherein the microcontroller permits the supply of the RF energy to a connected electrosurgical hand device when a subsequent generator failure is not detected.

## Claims

1. A method for performing an auto-verification and self-verification system of an electrosurgical generator prior to performing surgical procedure, the method comprising:
initiating a self-verification system test after determining a predetermined period of time has elapsed upon activation of the generator;
setting RF regulation modes and RF resolution settings after initiating the self-verification system test;
generating RF energy and directing RF output to a plurality of impedance loads within the generator;
measuring electrical characteristics of the RF output and analyzing the measured data;
determining whether the self-verification system test has been completed; and
recording self-verification system test completion timestamp upon completion of the self-verification system test.

2. The method of claim 1 further comprising repeating the setting, generating, measuring and analyzing steps until a completion of the self-verification system test is determined.

3. The method of claim 1 further comprising the step of initiating supply of RF energy to a connected electrosurgical hand device, if a generator failure is not detected.

4. The method of claim 1 further comprising the step of generating a system error if a generator failure is detected and a failure threshold is reached.

5. The method of claim 4 wherein the failure threshold is reached when the self-verification system test detects the generator failure for a number of consecutive times.

6. The method of claim 1 wherein the measuring step and the analyzing step are performed respectively by a feedback system and a microcontroller of the generator.

7. The method of claim 6 wherein the measuring step comprises measuring the electrical properties of the RF output across a plurality of channels from the feedback system and communicating the real and imaginary components thereof for the plurality of channels to the microcontroller.

8. The method of claim 7 wherein the analyzing step comprises receiving the real and imaginary components of the measured data, performing power calculations and comparing the measurement values of the feedback system channels for determining whether a generator failure exists.

9. The method of claim 8 wherein the generator failure exists if measurements values of the feedback system channels are not matching each other and/or are not within a certain tolerance across the plurality of RF regulation modes and RF resolution settings.

10. The method of claim 8 wherein the microcontroller initiates or halts supply of RF energy to a connected electrosurgical hand device based on the comparison results.

11. The method of claim 1 further comprising the step of storing results obtained from the measuring and analyzing steps into a memory of the generator.

12. The method of claim 1 wherein the plurality of RF regulation modes comprises one of voltage, current, power and/or phase regulation mode.

13. The method of claim 1 wherein the plurality of RF resolution settings comprises one of a low, medium or high voltage setting.

14. The method of claim 1 wherein the step of setting RF regulation modes and RF resolution settings further comprises setting a predetermined RF regulation value and activating appropriate relays for obtaining specific impedance load configuration.

15. The method of any one of the previous claims wherein the plurality of impedance loads are internal or integrated within the electrosurgical generator and are only used to perform the self-verification system test upon activation of the generator.
